(19) Europäisches Patentamt
European Patent Office
Office européen des brevets

(11) EP 0 614 905 B1

(12) **FASCICULE DE BREVET EUROPEEN**

(45) Date de publication et mention de la délivrance du brevet:
**11.08.1999 Bulletin 1999/32**

(51) Int Cl.[6]: **C07H 17/08**, A61K 31/70

(21) Numéro de dépôt: **94400491.0**

(22) Date de dépôt: **08.03.1994**

# (54) Nouveaux dérivés de l'érythromycine, leur procédé de préparation et leur application comme médicaments

Erythromycinderivate, ihr Verfahren zur Herstellung und ihre Verwendung als Arzneimittel

Erythromycin derivatives, their process of preparation and their application as medicaments

(84) Etats contractants désignés:
**AT BE CH DE DK ES FR GB GR IE IT LI LU NL PT SE**

(30) Priorité: **09.03.1993 FR 9302674**

(43) Date de publication de la demande:
**14.09.1994 Bulletin 1994/37**

(73) Titulaire: **HOECHST MARION ROUSSEL**
**92800 Puteaux (FR)**

(72) Inventeurs:
- **Agouridas, Constantin**
  **F-94130 Nogent sur Marne (FR)**
- **Benedetti, Yannick**
  **F-93110 Rosny sous Bois (FR)**
- **Chantot, Jean-François**
  **F-77410 Gressy en France (FR)**
- **Denis, Alexis**
  **F-75011 Paris (FR)**

(74) Mandataire: **Vieillefosse, Jean-Claude et al**
**Hoechst Marion Roussel**
**Département des Brevets**
**102, Route de Noisy**
**93235 Romainville Cédex (FR)**

(56) Documents cités:
**EP-A- 0 284 203**
**EP-A- 0 487 411**
**FR-A- 2 534 588**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 614 905 B1

## Description

La présente invention a pour objet de nouveaux dérivés de l'érythromycine, leur procédé de préparation et leur application comme médicaments.

On connaissait déjà des dérivés de la (R) (E) 9-O-(3-pipéridinyl)oxime de 3-dé((2,6-didéoxy-3-C-méthyl-3-O-méthyl alpha-L-ribohexopyranosyl)oxy)-6-O-méthyl-3-oxo-érythromycine (cf EP 487411).

L'invention a pour objet,1) Sous toutes leurs formes stéréoisomères possibles ainsi que leurs mélanges, les composés de formule (I) :

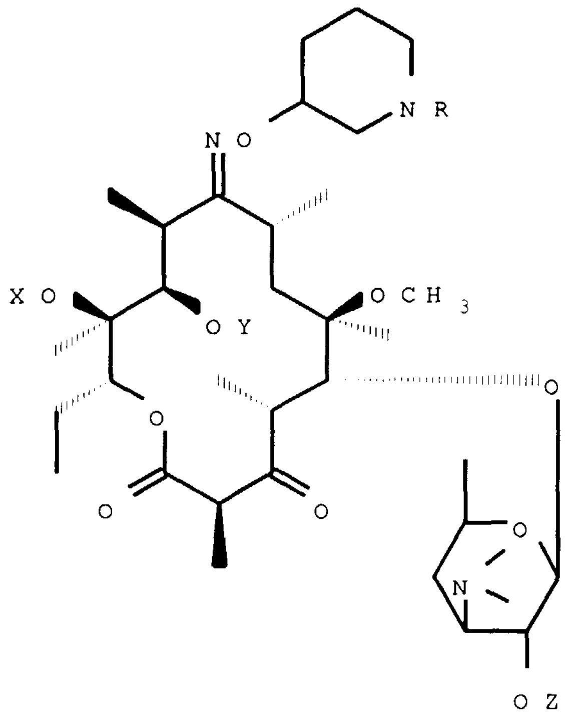

(I)

dans laquelle :

- X et Y représentent un atome d'hydrogène ou forment ensemble un radical :

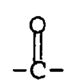

caractérisés en ce que

- R représente un radical :

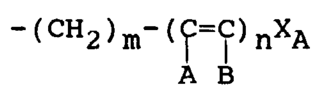

dans lequel m représente un nombre entier compris entre 0 et 20, n représente le nombre 0, 1, 2, ou 3, et ou bien A et B identiques ou différents représentent un atome d'hydrogène ou un atome d'halogène ou un radical alkyle ou aryle, renfermant jusqu'à 8 atomes de carbone, la géométrie de la double liaison étant E ou Z ou un mélange E + Z,

ou bien A et B forment une troisième liaison entre les atomes de carbone auxquels ils sont liés,

ou bien $X_A$ représente un radical phényle éventuellement substitué par un ou plusieurs radicaux choisis dans le groupe constitué par les radicaux carboxyle libre, salifié, estérifié ou amidifié, les radicaux hydroxyles, les atomes d'halogène, les radicaux $NO_2$, les radicaux C≡N, les radicaux alkyle, alkényle et alkynyle, O-alkyle, O-alkényle et O-alkynyle, S-alkyle, S-alkényle et S-alkynyle, SO-alkyle, SO-alkényle, SO-alkynyle, $SO_2$-alkyle, $SO_2$-alkényle, $SO_2$-alky-nyle, renfermant jusqu'à 12 atomes de carbone éventuellement substitués par un ou plusieurs atomes d'halogène, les radicaux aryle, O-aryle, S-aryle carbocycliques ou hétérocycliques renfermant jusqu'à 14 atomes de carbone, les radicaux :

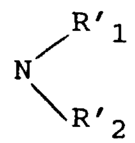

$R'_1$ et $R'_2$ identiques ou différents représentant un atome d'hydrogène ou un radical alkyle, renfermant jusqu'à 12 atomes de carbone, les radicaux aryles eux-mêmes éventuellement substitués par l'un des substituants indiqués ci-dessus comme substituants des radicaux aryles,

ou bien $X_A$ représente un radical :

$$-O-C(C_6H_5)_3$$

dans lequel le ou les radicaux phényle sont éventuellement substitués par un ou plusieurs des substituants indiqués précédemment,

ou bien $X_A$ représente un radical :

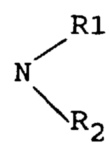

dans lequel $R_1$ et $R_2$ identiques ou différents représentent un atome d'hydrogène ou un radical alkyle renfermant jusqu'à 12 atomes de carbone, ainsi que leurs sels d'addition avec les acides.

Les composés de formule (I) peuvent exister sous toutes leurs formes stéréoisomères possibles, ainsi que leurs mélanges.

L'oxime en 9, par exemple peut être de géométrie E ou Z, et l'invention a pour objet les oximes E, les oximes Z ainsi que leurs mélanges E + Z.

Comme exemple de sels d'addition des présents dérivés avec les acides minéraux ou organiques, on peut citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et stéarique.

Lorsque $X_A$ représente un radical alkyle renfermant de 6 à 20 atomes de carbone, il s'agit de préférence d'un radical hexyle, heptyle, octyle, nonyle, décyle, undécyle, dodécyle, tridécyle, tétradécyle, pentadécyle ou hexadécyle, ainsi que de leurs isomères.

Dans la définition des substituants :

- l'halogène est de préférence le fluor ou le chlore ou le brome,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, propargyle,
- Dans le radical alkyle éventuellement interrompu par un ou plusieurs hétéroatomes, il s'agit de préférence de un ou plusieurs atomes d'oxygène.
- le radical aryle carbocyclique est de préférence le radical phényle ou naphtyle.

Par radical aryle hétérocyclique, on entend soit un radical hétéroaryle monocyclique à 5 ou 6 chainons comportant un ou plusieurs hétéroatomes , soit un système polycyclique condensé, chaque cycle comprenant 5 ou 6 chainons et le cas échéant un ou plusieurs hétéroatomes.

- le radical aryle hétérocyclique comporte un ou plusieurs hétéroatomes choisis de préférence parmi l'oxygène, le soufre et l'azote.
- le radical hétéroaryle monocyclique à 5 chaînons est de préférence le radical thiényle, furyle, pyrrolyle, thiazolyle, oxazolyle, imidazolyle, thiadiazolyle, pyrazolyle ou isoxazolyle,
- le radical hétéroaryle monocyclique à 6 chaînons est de préférence un radical pyridyle, pyrimidinyle, pyridazinyle ou pyrazinyle,
- le radical hétéroaryle polycyclique condensé peut être par exemple le radical indolyle, benzofuryle, benzothiényle ou quinoléinyle, ou le reste d'une base purine telle que l'adenine,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, propargyle, cyclobutyle, cyclopentyle ou cyclohexyle,
- le reste d'acide carboxylique est de préférence le reste acétyle, propionyle, butyryle, isobutyryle, n-valéryle, isovaléryle, tert-valéryle et pivalyle.

Parmi les composés préférés de l'invention, on peut citer les composés de formule (I) dans lesquels n représente le nombre 0 ou 1, ou encore ceux dans lesquels m représente un nombre entier compris entre 2 et 6 et ceux dans lesquels $X_A$ représente un radical phényle, éventuellement substitué par un ou plusieurs radicaux choisis dans le groupe constitué par les atomes d'halogène, les radicaux méthyles, trifluorométhyle, hydroxy, méthoxy, phényle ou phénoxy éventuellement substitués par un ou plusieurs atomes d'halogène.

Parmi ces composés, on peut citer tout particulièrement les composés dans lesquels R représente un radical $-(CH_2)_3Ar$, $-(CH_2)_4-Ar$ ou $-CH_2-CH=CH-Ar$, Ar représentant un radical phényle, éventuellement substitué, ainsi que leurs sels d'addition avec les acides.

Parmi les composés de l'invention, on peut également citer les composés de formule (I) dans lesquels $X_A$ représente le radical dodécylamino.

L'invention a plus particulièrement pour objet les composés dont la préparation est donnée ci-après dans la partie expérimentale et notamment les composés de l'exemple 1, celui de l'exemple 2, ainsi que ceux des exemples 4, 5, 21, 25, 26, 33, 37, 38, 42, 48, 51, 54, 57, 64, 65 et 66.

Les produits de formule générale (I) possèdent une très bonne activité antibiotique sur les bactéries gram$^{\oplus}$ telles que les staphylocoques, les streptocoques, les pneumocoques.

Les composés de l'invention peuvent donc être utilisés comme médicaments dans le traitement des infections à germes sensibles et, notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aigües primitives ou post-grippales, bronchopneumonies, suppuration pulmonaires, les streptococcies telles que les angines aigües, les otites, les sinusites, la scarlatine, les pneumococcies telles que les pneumonies, les bronchites ; la brucellose, la diphtérie, la gonococcie. Les produits de la présente invention sont également actifs contre les infections dues à des germes comme Haemophilus influenzae, Rickettsies, Mycoplasma pneumoniae, Chlamydia, Legionella, Ureaplasma, Toxoplasma, Listeria, Campylobacter et Méningocoque.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et notamment de médicaments antibiotiques, les produits préférés de formule (I) définis précédemment à savoir les produits des exemples 1, 2, 4, 5, 21, 25, 26, 33, 37, 38, 42, 48, 51, 54, 57, 64, 65 et 66 ainsi que leurs sels pharmaceutiquement acceptables.

L'invention a également pour objet les compositions pharmaceutiques renfermant comme principe actif au moins un des médicaments défini ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques,

les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1 ou le produit de l'exemple 2.

L'invention a également pour objet un procédé de préparation des composés de formule (I) caractérisé en ce que l'on soumet un composé de formule (II) :

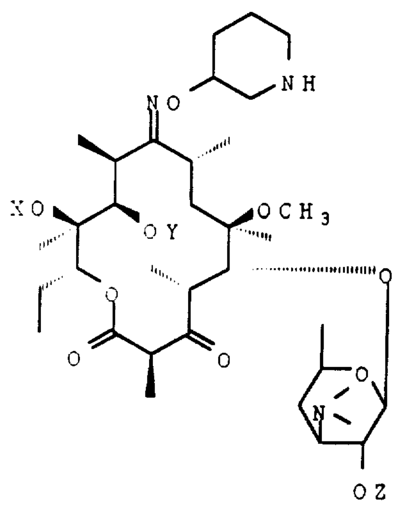

**(II)**

sous toutes ses formes stéréoisomères possibles ainsi que ses mélanges, dans laquelle X, Y et Z conservent leur signification précédente à l'action d'un composé de formule (III) :

$$RCHO \qquad (III)$$

dans laquelle R conserve la même signification que ci-dessus, pour obtenir le composé de formule (I) correspondant que l'on soumet, si désiré, à l'action d'un agent d'estérification de la fonction hydroxyle en 2' ou à l'action d'un acide pour en former le sel.

Dans un mode de réalisation préféré du procédé de l'invention, on fait réagir le composé de formule (II) et le composé de formule (III) en présence de $NaBH_3CN$, ou en présence d'hydrogène et d'un catalyseur adapté comme le palladium sur charbon actif.

L'estérification éventuelle en 2' est réalisée selon les procédés classiques. La salification éventuelle est réalisée au moyen d'acides selon les procédés classiques.

Les composés de formule (II) sous toutes leurs formes stéréoisomères ainsi que leurs mélanges sont des produits connus décrits dans la demande de brevet européen 0487411.

Les composés de formule (II) dans lesquels l'oxime en 9 est de géométrie E peuvent être obtenus par exemple par chromatographie à partir de mélanges de diastéréosiomères R + S au niveau du carbone en 3 de la pipéridine, ou peuvent également être obtenus en utilisant comme matière première la (S) 3-hydroxy pipéridine chirale obtenue par dédoublement à l'aide d'un acide chiral, selon le procédé décrit dans la demande de brevet européen 487411 (cf préparation de l'exemple 27).

Les composés de formule (III) sont des produits connus d'une façon générale qui peuvent être préparés selon le schéma réactionnel :

$$RCO_2H \rightarrow RCH_2OH \rightarrow RCHO$$

en utilisant les procédés classiques.

Les exemples suivants illustrent l'invention sans toutefois la limiter.

Les composés préparés ci-après sont préparés selon l'un des 3 modes opératoires généraux suivants :
Dans ce qui suit, on appelle produit A
1a (R) (E) 9-O-(3-pipéridinyl) oxime de 3-dé((2,6-didéoxy-3-C-méthyl 3-0-méthyl alpha-L-ribohexopyranosyl) oxy) 6-O-méthyl 3-oxo érythromycine.

Mode opératoire 1

On met en solution dans 4 ml de méthanol ou 3 ml de cyanure de méthyle + 1 ml de méthanol, 0,5 mM de produit A, 0,5 mM d'aldéhyde, on ajoute 0,1 ml d'acide acétique glacial, on agite de 5 à 10 mn et on ajoute en une fois 0,55 mM de $NaBH_3CN$.
On agite à température ambiante de 15 à 60 mn puis on concentre la solution sous pression réduite, on reprend avec 10 ml d'eau et 30 ml d'acétate d'éthyle, on amène à pH $\simeq$ 8 avec de la soude, on décante et extrait, on lave la phase aqueuse avec 10 ml d'acétate d'éthyle, puis on lave les phases organiques avec une solution saturée de chlorure de sodium. On sèche sur sulfate de magnésium. Les produits obtenus sont purifiés par chromatographie sur silice en éluant avec des mélanges du type acétate d'éthyle-triéthylamine ; éther isopropylique-triéthylamine-méthanol ; chlorure de méthylène-méthanol ; chlorure de méthylène-méthanol-ammoniaque ; chloroforme-méthanol-ammoniaque ; acétate d'éthyleméthanol ; acétate d'éthyle-méthanol-triéthylamine.

Mode opératoire 2

On met en solution dans 10 ml de méthanol, 0,4 mM de produit A, 0,45 mM d'aldéhyde, on ajoute 30 µl d'acide acétique glacial et 50 mg de palladium sur charbon actif, on agite sous 1,5 atm d'hydrogène pendant 2 à 24 h.
On filtre la solution sur clarcel, rince avec 20 ml de méthanol et concentre la solution. On purifie par chromatographie sur silice en éluant avec un des systèmes éluants du mode opératoire 1.

Mode opératoire 3

On met en solution dans 0,6 ml de cyanure de méthyle et 0,5 ml d'une solution de phosphate acide de sodium, 0,2 mM de produit A, 0,3 mM d'aldéhyde, on agite 5 mn, puis on ajoute 30 mg (0,48 mM) de $NaBH_3CN$, on ajuste le pH à - 4,5 en ajoutant au goutte-à-goutte, une solution d'acide chlorhydrique. La réaction est finie en 5 à 60 mn. On ajoute 5 ml d'eau, ajuste le pH à 8 avec de la soude et on extrait avec 2 x 10 ml de chloroforme, on sèche sur sulfate de magnésium les phases organiques.
On purifie par chromatographie sur silice en utilisant un des systèmes éluants du mode opératoire 1.

**EXEMPLE 1 : (R)(E) 9-O-(1-(3-phénylpropyl) 3-pipéridinyl) oxime de 3-dé((2,6-didéoxy-3-C-méthyl 3-O-méthyl alpha-L-ribohexopyranosyl) oxy) 6-0-méthyl 3-oxo érythromycine**

Mode opératoire 1
Spectre de masse : $MH^+ = 804^+$
RMN $CDCl_3$ ppm
2,25-2,75-2,98 (m) $\underline{CH_2}N$ ; 2,26 (s) $N\underline{CH_3}$ ; 2,74 (s) 6-$O\underline{CH_3}$ ; 3,69 (m) $H_8$ ; 3,86 (q) $H_2$ ; 4,04 (m) $NO\underline{CH}$ ; 5,17 (dd) $H_{13}$ ; 7,1 à 7,3 aromatiques.

**EXEMPLE 2 : (R)(E) 9-O-(1-dodécyl 3-pipéridinyl) oxime de 3-dé((2,6-didéoxy-3-C-méthyl 3-O-méthyl alpha-L-ribohexopyranosyl) oxy) 6-O-méthyl 3-oxo érythromycine,**

Mode opératoire 2
Spectre de masse : $MH^+ = 854^+$
RMN $CDCl_3$ ppm
1,26 les $(CH_2)_n$ ; 2,15 à 2,4-2,7-2,98 (m) $\underline{CH_2}N$ ; 2,26 (s) $N\underline{CH_3}$ ; 2,74 (s) 6-$O\underline{CH_3}$ ; 3,68 (m) $H_8$ ; 3,87 (q) $H_2$ ; 4,04 (m) $NO\underline{CH}$ ; 5,17 (dd) $H_{13}$.

**EXEMPLE 3 : (R)(E) 9-O-(1-octyl 3-pipéridinyl) oxime de 3-dé((2,6-didéoxy-3-C-méthyl 3-O-méthyl alpha-L-ribohexopyranosyl) oxy) 6-O-méthyl 3-oxo érythromycine,**

Mode opératoire 1
Spectre de masse : $MH^+ = 798^+$
RMN $CDCl_3$ ppm

1,22 à 1,33 $(CH_2)_n$ et 12$\underline{CH_3}$ ; 2,26 (s) N$\underline{CH_3}$ ; 2,74 (s) 6-O$\underline{CH_3}$ ; 2,2-2,7-2,98 les $\underline{CH_2}$N ; 3,68 (m) $H_8$ ; 3,86 (q) $H_2$ ; 4,04 (m) NOCH ; 5,17 (dd) $H_{13}$.

**EXEMPLE 4 : (R) (E) 9-O-(1-(3-(3-méthoxyphényl) propyl) 3- pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 834^+$
RMN $CDCl_3$ ppm
1,23 (s) 12$\underline{CH_3}$ ; 2,26 (s) N$\underline{CH_3}$ ; 2,74 (s) 6-O$\underline{CH_3}$ ; 2,3 à 2,6 les $\underline{CH_2}$N ; 3,68 (m) $H_8$ ; 3,80 (s) ΦO$\underline{CH_3}$ ; 3,87 (q) $H_2$ ; 4,04 (m) NO$\underline{CH}$ ; 5,17 (dd) $H_{13}$ ; 6,69-6,87-7,19 les aromatiques.

**EXEMPLE 5 : (R) (E) 9-O-(1-(4-triphénylméthoxy) butyl) 3-pipéridinyl) oxime de 3-dé((2,6-didéoxy-3-C-méthyl 6-O-méthyl alpha-L-ribohexopyranosyl) oxy) 6-O-méthyl 3-oxo érythromycine**

Mode opératoire 1
Spectre de masse : $MH^+ = 1000^+$
RMN $CDCl_3$ ppm
1,23 (s) 12$\underline{CH_3}$ ; 2,26 (s) N$\underline{CH_3}$ ; 2,1 à 2,4-2,97-2,72 les $\underline{CH_2}$N ; 3,06 $\underline{CH_2}$O ; 3,66 (m) $H_8$ ; 3,86 (q) $H_2$ ; 5,17 (dd) $H_{13}$ ; 7,17 à 7,30-7,44 (m) les aromatiques.

**EXEMPLE 6 : (R) (E) 9-O-(1-(4-hydroxybutyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 758^+$
RMN $CDCl_3$ ppm
1,23 (s) 12$\underline{CH_3}$ ; 2,26 (s) N$\underline{CH_3}$ ; 2,38 (sl) N$\underline{CH_2}$ ; 2,74 (s) 6-O$\underline{CH_3}$ ; 3,56 (sl) $\underline{CH_2}$OH ; 3,70 (m) $H_8$ ; 3,86 (q) $H_2$ ; 4,08 (m) NO$\underline{CH}$ .

**EXEMPLE 7 : (R) (E) 9-O-(1-(2-aminoéthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 729^+$
RMN $CDCl_3$ ppm
1,23 (s) 12$\underline{CH_3}$ ; 2,26 (s) N$\underline{CH_3}$ ; 2,4 (m)-2,76 (m) les $\underline{CH_2}$N ; 3,68 (m) $H_8$ ; 3,86 (q) $H_2$ ; 4,04 (m) NO$\underline{CH}$ ; 5,17 (dd) $H_{13}$.

**EXEMPLE 8 : (R) (E) 9-O-(1-(3-((3-(trifluorométhyl) phényl) oxy) phényl) méthyl) 3-pipéridinyl) oxime de 3-dé( (2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 936^+$
RMN $CDCl_3$ ppm
2,70 6-OMe ; 3,86 (q,J = 6 Hz) $H_2$ ; 3,67 (E) $H_8$ ; 2,27 $N(CH_3)_2$ ; 3,40 (d,J = 14)-3,459 (d,J = 14) N-$\underline{CH_2}$-Φ.

**EXEMPLE 9 : (R)(E) 9-O-(1-(2-phényléthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 790^+$
RMN $CDCl_3$ ppm
2,74 6-OMe ; 3,86 $H_2$ ; 3,70 (E) $H_8$ ; 7,15 à 7,35 Φ - ; 2,5 à 2,7-2,77-3,04 N-$CH_2$-$CH_2$- Φ et $CH_2$N.

**EXEMPLE 10 : (R) (E) 9-O-(1-(4-(4-méthoxyphényl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 848^+$
RMN $CDCl_3$ ppm

2,26 $N(CH_3)_2$ ; 2,74 (s) 6-OMe ; 3,68 $H_8$ ; 3,79 Φ-OMe ; 2,25 à 2,75-2,96 $CH_2N$ et $CH_2Φ$ ; 3,87 $H_2$.

**EXEMPLE 11 : (R)(E) 9-O-(1-(3-(1-(phénylméthyl) 1H-indol 4-yl) propyl 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 933^+$
RMN $CDCl_3$ ppm
2,26 $N(CH_3)_2$ ; 2,74 6-OMe ; 2,91 $CH_2$-Φ ; 3,68 $H_8$ ; 3,86 $H_2$ ; 4,06 axial N-O-CH : 5,32 $NCH_2Φ$ ; 6,58-6,92-7,05 à 7,30 les aromatiques.

**EXEMPLE 12 : (R) (E) 9-O-(1-(3-imidazolylpropyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 794^+$
RMN $CDCl_3$ ppm
2,26 $N(Me)_2$ ; 2,74 6-OMe ; 3,18 $H_2'$ ; 3,68 $H_8$ ; 3,89 $H_2$ ; 4,00 $CH_2$-N-C= 4,05 N-O-CH ; 7,46-6,92-7,05 imidazole.

**EXEMPLE 13 : (R) (E) 9-O-(1-(4-phénylbutyl) 3-pipéridimyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 818^+$
RMN $CDCl_3$ ppm
2,26 $N(Me)_2$ ; 2,62 $CH_2$-Φ ; 2,73 6-OMe ; 3,19 $H_2'$ ; 3,68 $H_8$ ; 3,89 $H_2$ ; 4,03 N-O-CH : 7,18-7,21 Aromatiques.

**EXEMPLE 14 : (R) (E) 9-O-(1-(((1,1'-biphényl) 4-yl) méthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 852^+$
RMN $CDCl_3$ ppm
2,73 (s) 6-OMe ; 3,69 (m) $H_8$ ; 3,86 (q) $H_2$ ; 3,47-3,63 (d) N-$CH_2$-Φ ; 4,09 (m) NO-CH axial.

**EXEMPLE 15 : (R) (E) 9-O-(1-((pentafluorophényl) méthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 866^+$
RMN $CDCl_3$ ppm
6-OMe ; 3,5 à 3,75 $H_8$ ; 3,86 (q) $H_2$ ; ~ 3,72 N-$CH_2$-Φ ; 4,03 (m) NO-CH axial.

**EXEMPLE 16 : (R) (E) 9-O-(1-((3-cyanophényl) méthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 3
Spectre de masse : $MH^+ = 800^+$
RMN $CDCl_3$ ppm
2,68 (s) 6-OMe ; 3,6 $H_8$ ; 3,87 (q) $H_2$ ; 3,43 (d) 3,60 N-$CH_2$-Φ ; 4,07 (m) NO-CH axial.

**EXEMPLE 17 : (R) (E) 9-O-(1-((1H-imidazol 2-yl) méthyl) 3- pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 766^+$
RMN $CDCl_3$ ppm
2,66 (s) 6-OMe ; 3,5 à 3,7 $H_8$ ; 3,87 (q) $H_2$ ; 4,09 (m) NO-CH.

**EXEMPLE 18 : (R)(E) 9-O-(1-((3-méthyl 2-thiényl) méthyl) 3-pipéridinyl) oxime de 3-dé((2,6-didéoxy-3-C-méthyl alpha-L-ribohexopyranosyl) oxy) 6-O-méthyl 3-oxo érythromycine**

Mode opératoire 1
Spectre de masse : $MH^+ = 796^+$
RMN $CDCl_3$ ppm
2,70 (s) 6-OMe ; 3,5 à 3,75 $H_8$ ; 3,86 (q) $H_2$ ; 4,06 (m) NO-CH axial ; 6,77 (d) 7,11 (d) thiophène $H_4$ $H_5$.

**EXEMPLE 19 : (R) (E) 9-O-(1-(3,3-diméthylbutyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 770^+$
RMN $CDCl_3$ ppm
2,74 (s) 6-OMe ; 3,69 (m) $H_8$ ; 3,86 (q) $H_2$ ; 4,04 (m) NO-CH.

**EXEMPLE 20 : (R)(E) 9-O-(1-(3-(méthylthio) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 774^+$
RMN $CDCl_3$ ppm
2,74 (s) 6-OMe ; 3,67 (m) $H_8$ ; 3,86 (q) $H_2$ ; 4,04 (m) NO-CH axial.

**EXEMPLE 21 : trans (R)(E) 9-O-(1-(3-phényl 2-propényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 802^+$
RMN $CDCl_3$ ppm
2,74 (s) 6-OMe ; 3,68 (m) $H_8$ ; 3,86 (q) $H_2$ ; 4,07 (m) NO-CH axial ; 6,24 (dt,J = 15,5 et 7) 6,49 (d,J = 15,5 éthylènique ΔE.

**EXEMPLE 22 : (R) (E) 9-O-(1-((2-méthoxyphényl) méthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 806^+$
RMN $CDCl_3$ ppm
2,68 (s) 6-OMe ; 3,5 à 3,75 $H_8$ ; 3,86 (q) $H_2$ ; 4,07 (m) NO-CH axial.

**EXEMPLE 23 : (R) (E) 9-O-(1-décyl 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 826^+$
RMN $CDCl_3$ ppm
2,74 (s) 6-OMe ; 3,68 (m) $H_8$ ; 3,86 (q) $H_2$ ; 4,04 (m) NO-CH axial.

**EXEMPLE 24 : (R) (E) 9-O-(1-(9-éthoxy 9-oxo nonyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 870^+$
RMN $CDCl_3$ ppm
2,74 (s) 6-OMe ; 3,68 (m) $H_8$ ; 3,86 (q) $H_2$ ; 4,03 NO-CH axial.

**EXEMPLE 25 : (R)(E) 9-O-(1-(3-(4-(trifluorométhyl) phényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 872^+$
RMN $CDCl_3$ ppm
1,23 (s) 12$\underline{CH_3}$ ; 2,26 (s) $N\underline{CH_3}$ ; 2,67 $\underline{CH_2}\Phi$ ; 2,74 (s) 6-O$\underline{CH_3}$; 3,67 (m) $H_8$ ; 3,87 (q) $H_2$ ; 4,04 (m) NO-$\underline{CH}$ ; 5,17 (dd) $H_{13}$ ; 7,30-7,53 les aromatiques.

**EXEMPLE 26 : (R) (E) 9-O-(1-(3-(3,4-difluorophényl) propyl) 3-pyridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 2
Spectre de masse : $MH^+ = 840^+$
RMN $CDCl_3$ ppm
1,23 (s) 12$\underline{CH_3}$ ; 2,28 (s) $N\underline{CH_3}$ ; 2,58 $CH_2\Phi$ ; 2,74 (s) 6-O$\underline{CH_3}$; 3,68 (m) $H_8$ ; 3,87 (q) $H_2$ ; 4,04 (m) NO-$\underline{CH}$ ; 5,17 (dd) $H_{13}$ ; 6,95-7,10 les aromatiques.

**EXEMPLE 27 : (R) (E) 9-O-(1-(2-phénoxyéthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythrolycine**

Mode opératoire 1
Spectre de masse : $MH^+ = 806^+$
RMN $CDCl_3$ ppm
1,22 (s) 12$\underline{CH_3}$ ; 2,26 (s) $N\underline{CH_3}$ ; 2,74 (s) 6-O$\underline{CH_3}$ ; 3,68 (m) $H_8$ ; 3,86 (q) $H_2$ ; 4,04 (m) NO-$\underline{CH}$ ; 4,08 (t) $\underline{CH_2}O\Phi$ ; 5,17 (dd) $H_{13}$ ; 6,85-7,25 les aromatiques.

**EXEMPLE 28 : (R) (E) 9-O-(1-(2-((phénylméthyl) amino) éthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 819^+$
RMN $CDCl_3$ ppm
1,23 (s) 12$\underline{CH_3}$ ; 2,26 (s) $N\underline{CH_3}$ ; 2,73 (s) 6-O$\underline{CH_3}$ ; 3,66 (m) 3,80 (s) $N\underline{CH_2}\Phi$ ; 4,01 (m) NO-$\underline{CH}$ ; 5,17 (dd) $H_{13}$ ; 7,27-7,32 (m) les aromatiques.

**EXEMPLE 29 : (E) (R) 9-O-(1-(2-(méthyl(phénylméthyl) amino) éthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 833^+$
RMN $CDCl_3$ ppm
1,22 (s) 12$\underline{CH_3}$ ; 2,22 (s) $N\underline{CH_3}$ ; 2,26 (s) $N\underline{CH_3}$ désosamine ; 2,76 (s) 6-O$\underline{CH_3}$ ; 3,51 (s) $N\underline{CH_2}\Phi$ ; 3,68 (m) $H_8$ ; 3,87 (q) $H_2$; 4,03 (m) NO-$\underline{CH}$ ; 7,20 à 7,40 les aromatiques.

**EXEMPLE 30 : (R) (E) 9-O-(1-(3-(4-méthoxyphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Mode opératoire 1
Spectre de masse : $MH^+ = 834^+$
RMN $CDCl_3$ ppm
1,23 (s) 12$\underline{CH_3}$ ; 2,26 (s) $N\underline{CH_3}$ ; 2,55 (m) $\underline{CH_2}\Phi$ ; 2,74 (s) 6-O$\underline{CH_3}$ ; 3,67 (m) $H_8$ ; 3,79 (s) $\Phi O\underline{CH_3}$ ; 3,86 (q) $H_2$ ; 4,04 (m) NO-$\underline{CH}$ ; 5,18 (dd) $H_{13}$ ; 6,82-7,10 les aromatiques.

**EXEMPLE 31 : (R) (E) 9-O-(1-(2-((phénylméthyl) ((phénylméthoxy) carbonyl) amino) éthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 953^+$
RMN $CDCl_3$ ppm
1,23 (s) 12Me ; 2,26 (s) $N\underline{CH_3}$ ; 2,71 (s) 6-O$\underline{CH_3}$ ; 2,92-3,30-3,37 (m) $N\underline{CH_2}$ ; 3,68 (m) $H_8$ ; 3,87 (q) $H_2$ ; 3,98 (m) NO$\underline{CH}$ ;

4,55 N$\underline{CH_2}$-$\Phi$ ; 7,2 à 7,40 les aromatiques.

**EXEMPLE 32 : (E)(R) 9-O-(1-((2-benzofuranyl) méthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 816^+$
RMN $CDCl_3$ ppm
1,22 : 12Me ; 1,35 : 6Me ; 2,27 : N-$(Me)_2$ ; 2,56 : $H_{10}$ ; 2,74: 6-OMe ; 3,19 : $H_2$' ; 3,65 : $H_8$ ; 3,87 : $H_2$ ; 4,11 : NO-CH ; 6,56 : benzofuranne $H_3$.

**EXEMPLE 33 : (E) (Z) 9-O-(1-(4-(4-méthylphényl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 832^+$
RMN $CDCl_3$ ppm
1,23 : 12Me ; 1,37 : 6Me ; 2,26 : N-$(Me)_2$ ; 2,57 : $H_{10}$ et $CH_2\Phi$ ; 2,73: 6-OMe ; 3,19 : $H_2$' ; 3,68 : $H_8$ ; 3,86 : $H_2$ ; 4,03 : NO-CH ; 7,07 :

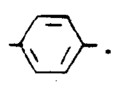

**EXEMPLE 34 : (E)(R) 9-O-(1-hexadécyl) 3-pipéridinyl) oxime de 3-dé(2,6-didéoxy-3-C-méthyl 3-O-méthyl alpha-L-ribohexopy ranosyl) oxy) 6-O-méthyl 3-oxo érythromycine**

Spectre de masse : $MH^+ = 910^+$
RMN $CDCl_3$ ppm
1,7-1,33 : 12Me ; 1,38 : 6Me ; 2,26 : N-$(Me)_2$ ; 2,56 : $H_{10}$ ; 2,74: 6-OMe ; 3,19 : $H_2$' ; 3,68 : $H_8$ ; 3,86 : $H_2$ ; 4,04 : NO-CH.

**EXEMPLE 35 : (E)(R) 9-O-(1-(2-(1-oxononylamino) éthyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

RMN $CDCl_3$ ppm
1,32 (s) : 6Me ; 2,17 (t) : $CH_2$-CO ; 2,26 (s) : $N(Me)_2$ ; 3,32 (m) : $CH_2NCO$ ; 3,67 (m) : $H_8$ ; 3,86 (q) : $H_2$ ; 4,02 (m): NO-CH ; 5,17 (dd) : $H_{13}$.

**EXEMPLE 36 : Trans (E)(R) 9-O-(1-((2-phényl 1-cyclopropyl) méthyl) 3-pipéridinyl) oxime de 3-((2,6-didéoxy-3-C-méthyl 3-O-méthyl alpha-L-ribohexopyranosyl) oxy) 6-O-méthyl 3-oxo érythromycine**

Spectre de masse : $MH^+ = 816^+$
RMN $CDCl_3$ ppm
1,22 (s) : 12Me ; 1,35 (s) : 6Me ; 2,26 (s) : N-$(Me)_2$ ; 3,67 : $H_8$ ; 3,86 (q) : $H_2$ ; 4,05 : NO-CH ; 5,17 (dd) : $H_{13}$ ; 7,04-7,23 : aromatiques.

**EXEMPLE 37 : (E) (R) 9-O-(1-(3-cyclohexylpropyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 810^+$
RMN $CDCl_3$ ppm
1,23 : 12Me ; 1,37 : 6Me ; 2,28 : N-$(Me)_2$ ; 2,57 : $H_{10}$ ; 2,73: 6-OMe ; 3,20 : $H_2$' ; 3,68 : $H_8$ ; 3,86 : $H_2$ ; 4,13 : N-OCH.

**EXEMPLE 38 : (E) (R) 9-O-(1-(3-(4-hydroxyphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

RMN $CDCl_3$ ppm
1,23 : 12Me ; 1,34 : 6Me ; 2,28 : N-$(Me)_2$ ; 2,75 : $H_{10}$ ; $CH_2\Phi$: 2,25 à 2,65 ; 2,66 : 6-OMe ; 3,19 : $H_2$' ; 3,65 : $H_8$ ; 3,88 : $H_2$ ; 4,07 : NO-CH ; 6,75-6,79 : -$\Phi$-O ; 3,33-4,37 : OH.

**EXEMPLE 39 : (E) (R) 9-O-(1-(3-(((phénylméthoxy)carbonyl) (phénylméthyl) amino) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 967,9^+$
RMN $CDCl_3$ ppm
1,22 (s) : 12Me ; 1,37 (s) : 6Me ; 2,26 (s) : N-$(Me)_2$ ; 2,72 (s) : 6-OMe ; 3,66 (m) : $H_8$ ; 3,85 (q) : $H_2$ ; 4,5 : $NCH_2Ar$ ; 5,16 : $OCH_2Ar$ ; 7,14 à 7,4 : aromatiques.

**EXEMPLE 40 : (E)(R) 9-O-(1-(3,3-diphényl 2-propényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 877^+$
RMN $CDCl_3$ ppm
1,22 (s) : 12Me ; 1,36 (s) : 6Me ; 2,26 (s) : N-$(Me)_2$ ; 2,70 (s) : 6-$OCH_3$ ; 3,66 (m) : $H_8$ ; 3,85 (q) : $H_2$ ; 4,04 (m) : NO-CH ; 6,17 (t) =CH ; 7,10 a 7,4 : aromatiques.

**EXEMPLE 41 : (E) (R) 9-O-(1-(3-(4-chlorophényl) 2(E)-propényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 836^+$
RMN $CDCl_3$ ppm
1,22 (s) : 12Me ; 1,38 (s) : 6Me ; 2,26 (s) : N-$(Me)_2$ ; 2,74 (s) : 6-OMe ; 3,68 (m) : $H_8$ ; 4,06 (m) : NO-CH ; 5,14 (dd) : $H_{13}$ ; 6,2 (dt) et 6,43 (d) : éthyléniques ; 7,2-7,3 : aromatiques.

**EXEMPLE 42 : (E) (R) 9-O-(1-undécyl 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 840^+$
RMN $CDCl_3$ ppm
1,26 (m) : $CH_2n$ et 12Me ; 1,38 (s) : 6Me ; 2,26 (s) : N-$(Me)_2$ ; 2,56 : $H_{10}$ ; 2,74 (s) : 6-OMe ; 3,68 (m) : $H_8$ ; 3,86 (q) : $H_2$ ; 4,03 (m) : NO-CH ; 5,17 (dd) : $H_{13}$.

**EXEMPLE 43 : (E)(R) 9-O-(1-(3 9-O-(1-(3-phénylméthyl) amino) propyl) 3 pipéridinyl) oxime de 3-dé((2,6-didéoxy-3-C-méthyl 3-O-méthyl alpha-L-ribohexopyranosyl) oxy) 6-O-méthyl 3-oxo érythromycine**

RMN $CDCl_3$ ppm
1,23 (s) : 12Me ; 1,37 (s) : 6Me ; 2,26 (s) : N-$(Me)_2$ ; 2,73 (s) : 6-OMe ; 3,67 (m) : $H_8$ ; 3,79 (AB) : $NCH_2Ar$ ; 3,86 (q) : $H_2$ ; 3,99 (m) : NO-CH ; 7,24-7,32 : aromatiques.

**EXEMPLE 44 : (E)(R) 9-O-(1-(8-phényloctyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 874^+$
RMN $CDCl_3$ ppm
1,25 : 12Me ; 1,38 : 6Me ; 2,26 : N-$(Me)_2$ ; 2,56 : $H_{10}$ ; 2,60: $\underline{CH_2}\Phi$ ; 2,74 : 6-OMe ; 3,18 : $H_2'$ ; 3,68 : $H_8$ ; 3,85 : $H_2$ ; 4,03 : N-OCH ; 7,16-7,26 : aromatiques.

**EXEMPLE 45 : (E)(R) 9-O-(1-(3-(4-nitrophényl) 2(E)-propényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 847^+$
RMN $CDCl_3$ ppm
1,22 : 12Me ; 1,37 : 6Me ; 2,26 : N-$(Me)_2$ ; 2,56 : $H_{10}$ ; 2,73: 6-OMe ; 3,05 à 3,20 : $H_2'$, $H_4$ ; 3,67 : $H_8$ ; 3,86 : $H_2$ ; 4,06 : N-OCH ; 7,5-8,17 : -$\Phi$-$NO_2$.

**EXEMPLE 46 : (E)(R) 9-O-(1-(3-(méthyloctylamino) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 869^+$
RMN $CDCl_3$ ppm
1,27 (s) : 12Me ; 1,38 (s) : 6Me ; 2,22 (s) : N-$(Me)_2$ 2,26 (s): N-$(Me)_2$ ; 3,68 (m) : $H_8$ ; 3,86 (q) : $H_2$ ; 4,03 (m) : NO-CH ; 5,17 (dd) : $H_{13}$.

**EXEMPLE 47 : (E)(R) 9-O-(1-(3-((phénylméthyl) (4-phényl 1-oxobutyl) amino) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 979^+$
RMN $CDCl_3$ ppm
2,26 (s) : N-$(Me)_2$ ; 3,68 (m) : $H_8$ ; 3,98 (m) : NO-CH ; 4,48-4,6 (AB) : $NCH_2Ar$ ; 5,16 (dd) : $H_{13}$ ; 7,05-7,4 : aromatiques.

**EXEMPLE 48 : (E) (R) 9-O-(1-(3-(1,1-biphényl 4-yl) 2(E)-propényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 878^+$
RMN $CDCl_3$ ppm
1,22 (s) : 12Me ; 1,38 (s) : 6Me ; 2,25 (s) : N-$(Me)_2$ ; 2,76 (s) : 6-OMe ; 3,69 (m) : $H_8$ ; 3,86 (q) : $H_2$ ; 5,16 (dd) $H_{13}$ ; 6,29-6,53 : éthyléniques ; 7,3-7,6 : aromatiques.

**EXEMPLE 49 : (E) (R) 9-O-(1-(3-(méthyl (phénylméthyl) amino) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 847^+$
RMN $CDCl_3$ ppm
1,25 (s) : 12Me ; 1,38 (s) : 6Me ; 2,18 (s) NMe ; 2,25 (s) : N-$(Me)_2$ ; 2,74 (s) : 6-OMe ; 3,47 (s) : $CH_2Ar$ ; 3,68 (m) : $H_8$; 3,86 (q) : $H_2$ ; 5,17 : $H_{13}$; 7,24-7,3 : aromatiques.

**EXEMPLE 50 : (E) (R) 9-O-(1-(3-(4-phényl 1H-imidazol-1-yl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 870^+$
RMN $CDCl_3$ ppm
1,23 (s) : 12Me ; 1,38 (s) : 6Me ; 2,25 (s) : N-$(Me)_2$ ; 3,68 (m) : $H_8$ ; 3,86 (q) : $H_2$ ; 4,05 (m) : NO-CH ; 5,14 (dd) : $H_{13}$; 7,2-7,49 : H imidazole ; 7,22-7,36-7,76 : aromatiques.

**EXEMPLE 51 : (E) (R) 9-O-(1-(3-(4-phénoxyphényl) 2(E)-propényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 895^+$
RMN $CDCl_3$ ppm
1,22 (s) : 12Me ; 1,38 (s) : 6Me ; 2,27 (s) : N-$(Me)_2$ ; 2,74 (s) : 6-OMe ; 3,69 (m) : $H_8$ ; 3,86 (q) : $H_2$ ; 4,02 (m) : NO-CH ; 5,15 (dd) : $H_{13}$ ; 6,15 et 6,46 (J=16Hz) : =CH ; 6,9 à 7,4 : aromatiques.

**EXEMPLE 52 : (E) (R) 9-O-(1-(3-(4-butoxyphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 876,7^+$
RMN $CDCl_3$ ppm
1,23 : 12Me ; 1,37 : 6Me ; 2,26 : N-$(Me)_2$ ; 2,30-2,60 : $H_{10}$ ; 2,74 : 6-OMe ; 3,70 : $H_2$' ; 3,68 : $H_8$ ; 3,87 : $H_2$ ; 4,05 : NO-CH ; 3,95 : -Φ-O-$CH_2$ ; 6,85-7,08 : -Φ-.

**EXEMPLE 53 : (E) (R) 9-O-(1-(3-((((1,1'-biphényl-4-yl) méthyl) amino) propyl)3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 909^+$
RMN $CDCl_3$ ppm
1,23 (s) : 12Me ; 1,38 (s) : 6Me ; 2,26 (s) : $N-(Me)_2$ ; 2,74 (s) : 6-OMe ; 3,45 (m) : $H_8$ ; 3,83 (s) : $CH_2Ar$ ; 3,86 : $H_2$ ; 4,02 (m) : NO-CH ; 5,17 (dd) : $H_{13}$ ; 7,3-7,6 : aromatiques.

**EXEMPLE 54 : (E) (R) 9-O-(1-(3-(dodécylamino) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 911^+$
RMN $CDCl_3$ ppm
0,96-1,14 : $CH_3-(CH)_n$ ; 1,37 (s) : 6Me ; 2,3 (s) : $N-(Me)_2$ ; 2,76 (s) : 6-OMe ; 3,68 (m) : $H_8$ ; 3,86 (q) : $H_2$ ; 5,17 (dd): $H_{13}$.

**EXEMPLE 55 : (E) (R) 9-O-(1-(4-(2,4,6-triméthylphényl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 860^+$
RMN $CDCl_3$ ppm
1,22 : 12Me ; 1,37 : 6Me ; 2,26 : $N-(Me)_2$ ; 2,56 : $H_{10}$ ; 3,18 : $H_2'$ ; 3,68 : $H_8$ ; 3,86 : $H_2$ ; 4,04 : N-OCH ; 7,4 à 7,7: -Φ-.

**EXEMPLE 56 : (E) (R) 9-O-(1-(5-phénylpentyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

RMN $CDCl_3$ ppm
1,23 : 12Me ; 1,38 : 6Me ; 2,26 : $N-(Me)_2$ ; 2,58 : $H_{10}$ ; 2,61: $CH_2\Phi$ ; 2,74: 6-OMe ; 3,18 : $H_2'$ ; 3,67 : $H_8$ ; 3,85 : $H_2$; 4,04 : N-OCH ; 7,15 à 7,35 : aromatiques.

**EXEMPLE 57 : (E) (R) 9-O-(1-(3,7,11-triméthyl 2(E),6(E),10-dodécatriényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 890^+$
RMN $CDCl_3$ ppm
1,22 (s) : 12Me ; 1,30 (s) : 6Me ; 1,6-1,62 : $CH_3$ vinyliques; 2,26 (s) : $N-(Me)_2$ ; 3,68 (m) : $H_8$ ; 3,86 (q) : $H_2$ ; 4,05 (m): NO-CH ; 5,05-5,25 : H vinyliques.

**EXEMPLE 58 : (E) (R) 9-O-(1-(3-(3,4,5-triméthoxyphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 894^+$
RMN $CDCl_3$ ppm
1,23 : 12Me ; 1,38 : 6Me ; 2,26 : $N-(Me)_2$ ; 2,39 : $H_{10}$ ; 2,74: 6-OMe ; 3,18 : $H_2'$ ; 3,68 : $H_8$ ; 3,8 : $H_2$ ; 4,05 : NO-CH ; 6,41 : aromatiques.

**EXEMPLE 59 : (E) (R) 9-O-(1-(5-((2-méthoxyéthoxy) méthoxy) pentyl) 3-pipéridinyl) oxime de 3-dé((2,6-dideoxy-3-C-méthyl 3-O-méthyl alpha-L-ribohexopyranosyl) oxy) 6-O-méthyl 3-oxo érythromycine**

RMN $CDCl_3$ ppm
1,38 (s) : 6Me ; 2,26 (s) : $N-(Me)_2$ ; 2,76 (s) : 6-OMe ; 3,52-3,71 : $OCH_2CH_2O$ ; 3,82 (q) : $H_2$ ; 4,09 : NO-CH ; 4,72 : $OCH_2O$ : 5,17 (dd) : $H_{13}$.

**EXEMPLE 60 : (E) (R) 9-O-(1-(6-phénylhexyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 846^+$
RMN $CDCl_3$ ppm
1,23 : 12Me ; 1,38 : 6Me ; 2,26 : $N-(Me)_2$ ; 2,60 : $CH_2-\Phi$ ; 2,74: 6-OMe ; 3,19 : $H_2'$ ; 3,69 : $H_8$ ; 3,87 : $H_2$ ; 4,04 : N-OCH ;

7,10 à 7,30 : aromatiques.

**EXEMPLE 61 : (E) (R) 9-O-(1-(6-((4,6-diméthyl 2-pyrimidinyl) thio) héxyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 908^+$
RMN $CDCl_3$ ppm
1,23 (s) : 12Me ; 1,38 (s) : 6Me ; 2,26 (s) : N-$(Me)_2$ ; 2,39 (s) : $CH_3$ aromatiques ; 2,7 (s) : 6-OMe ; 3,68 (m) : $H_8$ ; 3,86 (q) : $H_2$ ; 4,04 : NO-CH ; 5,17 (dd) : $H_{13}$ ; 6,67 : aromatiques.

**EXEMPLE 62 : (E) (R) 9-O-(1-(5-((4,4,5,5,5-pentafluoropentyl) sulfonyl) pentyl) 3-pipéridinyl) oxime de 3-dé((2,6-didéoxy-3-C-méthyl 3-C-méthyl alpha-L-ribohexopyranosyl) oxy) 6-O-méthyl 3-oxo érythromycine**

RMN $CDCl_3$ ppm
1,23 (s) : 12Me ; 1,38 (s) : 6Me ; 2,26 (m) : N-$(Me)_2$ ; 2,76 (s) : 6-OMe ; 2,97-3,10 : $CH_2SO_2$ ; 3,67 (m) : $H_8$ ; 3,87 (q) : $H_2$ ; 4,02 : NO-CH ; 5,16 (dd) : $H_{13}$.

**EXEMPLE 63 : (E) (R) 9-O-(1-tétradécyl 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 882,8^+$
RMN $CDCl_3$ ppm
1,25 : 12Me ; 1,38 : 6Me ; 2,26 : N-$(Me)_2$ ; 2,56 : $H_{10}$ ; 2,74: 6-OMe ; 3,19 : $H_2'$ ; 3,68 : $H_8$ ; 3,86 : $H_2$ ; 4,04 : NO-CH.

**EXEMPLE 64 : (R) (E) 9-O-(1-(4-(1,1'-biphényl 4-yl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 894^+$
RMN $CDCl_3$ ppm
1,23 (s) : 12Me ; 1,39 (s) : 6Me ; 2,66 (t) : $CH_2Ar$ ; 3,68 (m) : $H_8$ ; 3,86 (q) : $H_2$ ; 4,05 (m) : NO-CH ; 5,17 : $H_{13}$ ; 7,25-7,59 : aromatiques.

**EXEMPLE 65 : (R)(E) 9-O-(1-(3-(4-phénoxyphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 896^+$
RMN $CDCl_3$ ppm
1,22 (s) : 12Me ; 1,38 (s) : 6Me ; 2,26 (s) : N-$(Me)_2$ ; 2,6 : $CH_2Ar$ ; 2,76 (s) : 6-OMe ; 3,86 (q) : $H_2$ ; 4,05 (m) : NO-CH ; 5,17 (dd) : $H_{13}$ ; 6,85-7,9 : aromatiques.

**EXEMPLE 66 : (R)(E) 9-O-(1-(4-(3-(3,5-dichlorophénoxy) phényl) butyl) 3-pipéridinyl) oxime de 3-((2,6-didéoxy-3-C-méthyl 3-O-méthyl alpha-L-ribohexopyranosyl) oxy) 6-O-méthyl 3-oxo érythromycine**

Spectre de masse : $MH^+ = 978^+$
RMN $CDCl_3$ ppm
1,23 (s) : 12Me ; 1,38 (s) : 6Me ; 2,26 (s) : N-$(Me)_2$ ; 2,76 (s) : 6-OMe ; 3,67 (m) : $H_8$ ; 3,86 (q) : $H_2$ ; 4,03 (m) : NO-CH ; 5,18 (dd) : $H_{13}$ ; 6,85 (d)-7,05 (t)-6,82-7,29-7,03 : aromatiques.

**EXEMPLE 67 : (R)(E) 9-O-(1-(6-((4-méthylphényl) thio) hexyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 892^+$
RMN $CDCl_3$ ppm
1,22 : 12Me ; 1,38 : 6Me ; 2,26 : N-$(Me)_2$ ; 2,31 : $CH_3$-Φ ; 2,56 : $H_{10}$ ; 2,74: 60Me ; 2,87 : S-$CH_2$ ; 3,18 : $H_2'$ ; 3,69 : $H_8$ ; 3,86 : $H_2$ ; 4,03 : N-$OCH_2$ ; 7,09 et 7,24 : -Φ-S.

**EXEMPLE 68 : (E)(R) 9-O-(1-(4-(4-butylphényl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 874,6^+$
RMN $CDCl_3$ ppm
1,23 : 12Me ; 1,38 : 6Me ; 2,26 : $N-(Me)_2$ ; 2,58 : $H_{10}$ et $CH_2\Phi$ ; 2,74: 6-OMe ; 3,19 : $H_2'$ ; 3,68 : $H_8$ ; 3,87 : $H_2$ ; 4,03 : NO-CH ; 7,08 : -Φ-.

**EXEMPLE 69 : (R)(E) 9-O-(1-(6-(4-chlorophénoxy) hexyl) 3pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 896^+$
RMN $CDCl_3$ ppm
1,24 : 12Me ; 1,38 : 6Me ; 2,26 : $N-(Me)_2$ ; 2,56 : $H_{10}$ ; 2,74: 6-OMe ; 3,68 : $H_8$ ; 3,86 : $H_2$ ; 4,04 : =N-O-CH axial ; 6,82 : -Φ-Cl.

**EXEMPLE 70 : (E) (R) 9-O-(1-(3-((4-méthylphényl) thio) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 850^+$
RMN $CDCl_3$ ppm
1,29 (s) : 12Me ; 1,37 (s) : 6Me ; 2,28 (s) : $N-(Me)_2$ ; 2,32 (s) : $CH_3Ar$ ; 2,99 : $CH_2$-S ; 3,67 (m) : $H_8$ ; 3,86 (q) : $H_2$ ; 5,17 (dd) : $H_{13}$ ; 7,09-7,25 : aromatiques.

**EXEMPLE 71 : (E) (R) 9-O-(1-(4-(4-(1H-pyrrol 1-yl) phényl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 883^+$
RMN $CDCl_3$ ppm
1,23 (s) : 12Me ; 1,39 (s) : 6Me ; 2,26 (s) : $N-(Me)_2$ ; 2,64: $CH_2Ar$ ; 2,76 (s) : 6-OMe ; 3,68 (m) : $H_8$ ; 3,86 (q) : $H_2$ ; 4,04 (m) : NO-CH ; 5,18 (dd) : $H_{13}$ ; 6,33-7,07 : pyrrole ; 7,22-7,31 : phényle.

**EXEMPLE 72 : (E) (R) 9-O-(1-(4-(5-butyl 2-pyridinyl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 875^+$
RMN $CDCl_3$ ppm
1,23 (s) : 12Me ; 1,38 (s) : 6Me ; 2,26 (s) : $N-(Me)_2$ ; 2,57 (t) et 2,76 (t) : $CH_2$ benzylique ; 3,68 (m) : $H_8$ ; 3,86 (q) : $H_2$ ; 5,17 (dd) : $H_{13}$ ; 7,06-7,4-8,33 : pyridine.

**EXEMPLE 73 : (E) (R) 9-O-(1-(3-(phénylméthoxy) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 832^+$
RMN $CDCl_3$ ppm
1,23 : 12Me ; 1,38 : 6Me ; 2,26 : $N-(Me)_2$ ; 2,56 : $H_{10}$ ; 2,73: 6-OMe ; 3,18 : $H_2'$ ; 3,50 : OCH ; 3,68 : $H_8$ ; 3,86 : $H_2$; 4,03 : N-O-CH axial ; 4,5 : O-$\underline{CH_2}$-Φ ; 7,25 à 7,40 : aromatiques

**EXEMPLE 74 : (E)(R) 9-O-(1-(3-(4-phénylcyclohexyl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 886^+$
RMN $CDCl_3$ ppm
1,23 : 12Me ; 1,38 : 6Me ; 2,26 : $N-(Me)_2$ ; 2,57 : $H_{10}$ ; 3,18 : $H_2'$ ; 3,68 : $H_8$ ; 3,86 : $H_2$ ; 4,05 : N-O-CH axial ; 7,14 à 7,30 : aromatiques.

**<u>EXEMPLE 75</u> : (E)(R) 9-O-(1-(4-(4-(4-pyridinyl) phényl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 895^+$
<u>RMN $CDCl_3$ ppm</u>
1,23 : 12Me ; 1,38 : 6Me ; 2,27 : N-$(Me)_2$ ; 2,58 : $H_{10}$ ; 2,69: $CH_2\Phi$ ; 2,75: 6-OMe ; 3,20 : $H_2'$ ; 3,67 : $H_8$ ; 3,87 : $H_2$; 4,06 : NOCH ; 7,30-7,57 : phényle ; 7,51-8,64 : pyridine.

**<u>EXEMPLE 76</u> : (E) (R) 9-O-(1-(3-(4-méthylphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 158, 818^+$
<u>RMN $CDCl_3$ ppm</u>
2,31 : $CH_3\Phi$ ; 2,57 : $CH_2\Phi$ ; 3,67 : $H_8$ ; 4,04 : NO-CH< ;7,08 phényle.

**<u>EXEMPLE 77</u> : (E)(R) 9-O-(1-(3-(3-hydroxyphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 158, 820^+$
<u>RMN $CDCl_3$ ppm</u>
2,3 à 2,7 : $CH_2\Phi$ ; 3,65 : $H_8$ ; 4,06 : NO-CH< ; 6,67 (3H)-7,11 (1H) : phényle.

**<u>EXEMPLE 78</u> : (E)(R) 9-O-(1-(4-(4-hydroxyphényl) butyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 834, 840^+$
<u>RMN $CDCl_3$ ppm</u>
3,63 : $H_8$ ; 4,00 : NO-CH< ; 6,80-7,00 : phényle.

**<u>EXEMPLE 79</u> : 11,12-carbonate cyclique de (E)(R) 9-O-(1-(3-(4-hydroxyphényl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

Spectre de masse : $MH^+ = 846^+$
<u>RMN $CDCl_3$ ppm</u>
1,38 (s)-1,55(s) : 6Me ; 2,26 (s) : $N(Me)_2$ ; 2,62 (s) 6-OMe ; 3,67 (m) : $H_8$ (isomère E) ; 3,83 (q) : $H_2$ 4,09 : NO-CH< ; 5,05 (dd) : $H_{13}$ ; 6,76-7,05 : hydroxyphényle.

**<u>EXEMPLE 80</u> : (E)(R) 9-O-(1-(3-(4-quinoléinyl) 2(E)-propényl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

**<u>EXEMPLE 81</u> : (E)(R) 9-O-(1-(3-(4-quinoléinyl) propyl) 3-pipéridinyl) oxime de 3-dé((2,6-didéoxy-3-C-méthyl 3-O-méthyl alpha-L-ribohexopyranosyl) oxy) 6-O-méthyl 3-oxo érythromycine**

**<u>EXEMPLES DE COMPOSITIONS PHARMACEUTIQUES</u>**

On a préparé des comprimés renfermant :

| | |
|---|---|
| Produit de l'exemple 1 | 150 mg |
| Excipient q.s.p | 1 g |

Détail de l'excipient : amidon, talc, stéarate de magnésium.

| | |
|---|---|
| Produit de l'exemple 2 | 150 mg |
| Excipient q.s.p | 1 g |

Détail de l'excipient : amidon, talc, stéarate de magnésium.

| | |
|---|---|
| Produit de l'exemple 4 | 150 mg |

Excipient q.s.p 1 g

Détail de l'excipient : amidon, talc, stéarate de magnésium.

## ETUDE PHARMACOLOGIOUE DES PRODUITS DE L'INVENTION

### A) Activité in vitro

Méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/$cm^3$.

Les résultats suivants ont été obtenus (lecture après 24 heures).

| Produit de l'exemple 1 | |
|---|---|
| Staphylococcus aureus 011UC4 | 0,3 |
| Staphylococcus aureus 011HT17 | 0,3 |
| Staphylococcus aureus 011G025I | 1,2 |
| Staphylococcus epidermidis 012GO11C | 0,3 |
| Streptococcus pyogenes groupe A 02A1UC1 | 0,08 |
| Streptococcus agalactiae groupe B 02B1HT1 | ≤ 0,02 |
| Streptococcus sp groupe C 02C0CB3 | 0,08 |
| Streptococcus faecalis groupe D 02D2UC1 | 0,08 |
| Streptococcus faecium groupe D 02D3HT1 | 0,08 |
| Streptococcus sp groupe G 02G0Gr5 | 0,04 |
| Streptococcus mitis 02MitCB1 | ≤ 0,02 |
| Streptococcus agalactiae groupe B 02B1SJ1 | 0,3 |
| Streptococcus sp groupe C 02C0CB1 | 0,15 |
| Streptococcus faecalis groupe D 02D2Gr8 | 1,2 |
| Streptococcus pneumoniae 032UC1 | 0,08 |
| Streptococcus pneumoniae 030SJ1 | 1,2 |
| Streptococcus pneumoniae 030SJ5 | 0,3 |
| Haemophilus influenzae 351HT3 | 5 |
| Haemophilus influenzae 351CB12 | 0,6 |
| Haemophilus influenzae 351CA1 | 2,5 |

| Produit de l'exemple 2 | |
|---|---|
| Staphylococcus aureus 011UC4 | 1,2 |
| Staphylococcus aureus 011HT17 | 1,2 |
| Staphylococcus aureus 011B18C | 5 |
| Staphylococcus aureus 011GR12C | 5 |
| Staphylococcus aureus 011G025I | 2,5 |
| Staphylococcus epidermidis 012GO11C | 1,2 |
| Staphylococcus aureus 011CB20 | 2,5 |
| Staphylococcus epidermidis 012GO40 | 2,5 |
| Streptococcus pyogenes groupe A 02A1UC1 | 0,3 |
| Streptococcus agalactiae groupe B 02B1HT1 | 1,2 |
| Streptococcus sp groupe C 02C0CB3 | 1,2 |

(suite)

| Produit de l'exemple 2 | |
|---|---|
| Streptococcus faecalis groupe D 02D2UC1 | 0,3 |
| Streptococcus faecium groupe D 02D3HT1 | 0,3 |
| Streptococcus sp groupe G 02G0GR5 | 1,2 |
| Streptococcus mitis 02MitCB1 | 1,2 |
| Streptococcus pyogenes groupe A 02A1SJ1 | 1,2 |
| Streptococcus agalactiae groupe B 02B1SJ1 | 1,2 |
| Streptococcus sp groupe C 02C0CB1 | 0,6 |
| Streptococcus faecalis groupe D 02D2Gr8 | 2,5 |
| Streptococcus faecalis groupe D 02D2DU15 | 2,5 |
| Streptococcus sp groupe G 02G0GR4 | 1,2 |
| Streptococcus sanguis 02sgGR10 | 2,5 |
| Streptococcus mitis 02MitGR16 | 1,2 |
| Streptococcus pneumoniae 032UC1 | 1,2 |
| Streptococcus pneumoniae 030SJ1 | 1,2 |
| Streptococcus pneumoniae 030SJ5 | 2,5 |
| Haemophilus influenzae 351CB12 | 5 |

| Produit de l'exemple 4 | |
|---|---|
| Staphylococcus aureus 011UC4 | 0,3 |
| Staphylococcus aureus 011UC4 + sérum | 0,3 |
| Staphylococcus epidermidis 012GO11i | 1,2 |
| Streptococcus pyogenes groupe A 02A1UC1 | 0,08 |
| Streptococcus agalactiae groupe B 02B1HT1 | < 0,02 |
| Streptococcus faecalis 02D2UC1 | 0,08 |
| Streptococcus faecium groupe D 02D3HT1 | 0,08 |
| Streptococcus groupe G 02G0Gr5 | 0,08 |
| Streptococcus mitis 02MitCB1 | < 0,02 |
| Streptococcus agalactiae 02B1SJ1c | 0,3 |
| Streptococcus sanguis 02SgGR10i | 0,08 |
| Streptococcus mitis 02MitGR16i | 0,08 |
| Streptococcus pneumoniae 032UC1 | 0,3 |
| Streptococcus pneumoniae 030GR20 | 0,6 |
| Streptococcus pneumoniae 030SJ5i | 0,15 |
| Streptococcus pneumoniae 030R01 | 0,3 |
| Streptococcus pneumoniae 030SJ1c | 2,5 |

| Produit de l'exemple 5 | |
|---|---|
| Staphylococcus aureus 011UC4 | 2,5 |
| Staphylococcus aureus O11G025i | 5 |
| Staphylococcus aureus O11CB20c | 5 |
| Staphylococcus epidermidis 012GO40c | 5 |
| Streptococcus pyogenes groupe A 02A1UC1 | 0,3 |
| Streptococcus agalactiae groupe B 02B1HT1 | 0,08 |
| Streptococcus faecalis 02D2UC1 | 0,3 |
| Streptococcus faecium groupe D 02D3HT1 | 0,3 |
| Streptococcus groupe G 02G0Gr5 | 0,6 |

(suite)

| Produit de l'exemple 5 | |
|---|---|
| Streptococcus mitis 02MitCB1 | 0,15 |
| Streptococcus pyogenes 02A1SJ1c | 2,5 |
| Streptococcus agalactiae 02B1SJ1c | 2,5 |
| Streptococcus faecalis grpe D 02D2DU15c | 2,5 |
| Streptococcus sanguis 02SgGR10i | 0,3 |
| Streptococcus mitis 02MitGR16i | 0,6 |
| Streptococcus pneumoniae 032UC1 | 0,3 |
| Streptococcus pneumoniae 030GR20 | 0,3 |
| Streptococcus pneumoniae 030SJ5i | 1,2 |
| Streptococcus pneumoniae 030cr18 | 2,5 |
| Streptococcus pneumoniae 030PW23 | 2,5 |
| Streptococcus pneumoniae 030R01 | 1,2 |

| Produit de l'exemple 21 | |
|---|---|
| Staphylococcus aureus 011UC4 | 0,3 |
| Staphylococcus aureus 011UC4 + sérum | 0,15 |
| Staphylococcus aureus 011G025i | 1,2 |
| Staphylococcus epidermidis 012GO11i | 0,3 |
| Streptococcus pyogenes groupe A 02A1UC1 | 0,15 |
| Streptococcus agalactiae groupe B 02B1HT1 | 0,04 |
| Streptococcus faecalis 02D2UC1 | 0,15 |
| Streptococcus faecium groupe D 02D3HT1 | 0,15 |
| Streptococcus groupe G 02G0Gr5 | 0,15 |
| Streptococcus agalactiae 02B1SJ1c | 0,6 |
| Streptococcus sanguis 02SgGR10i | 0,8 |
| Streptococcus mitis 02MitGR16i | 0,15 |
| Streptococcus pneumoniae 032UC1 | 0,04 |
| Streptococcus pneumoniae 030GR20 | 0,08 |
| Streptococcus pneumoniae 030SJ5i | 0,3 |
| Streptococcus pneumoniae 030cr18 | 0,6 |
| Streptococcus pneumoniae 030PW23 | 2,5 |
| Streptococcus pneumoniae 030R01 | 0,3 |
| Haemophilus influenzae 351HT3 | 5 |
| Haemophilus influenzae 351CB12 | 0,6 |
| Haemophilus influenzae 351CA1 | 2,5 |

| Produit de l'exemple 26 | |
|---|---|
| Staphylococcus aureus 011UC4 | 0,3 |
| Staphylococcus aureus 011UC4 + sérum | 0,6 |
| Staphylococcus aureus 011G025i | 2,5 |
| Staphylococcus epidermidis 012GO11i | 0,6 |
| Streptococcus pyogenes groupe A 02A1UC1 | 0,08 |
| Streptococcus agalactiae groupe B 02B1HT1 | < 0,02 |
| Streptococcus faecalis 02D2UC1 | 0,08 |
| Streptococcus faecium groupe D 02D3HT1 | 0,08 |
| Streptococcus groupe G 02G0Gr5 | 0,08 |

(suite)

| Produit de l'exemple 26 | |
|---|---|
| Streptococcus mitis 02MitCB1 | 0,04 |
| Streptococcus agalactiae 02B1SJ1c | 0,6 |
| Streptococcus sanguis 02SgGR10i | 0,3 |
| Streptococcus pneumoniae 032UC1 | 0,3 |
| Streptococcus pneumoniae 030GR20 | 0,04 |
| Streptococcus pneumoniae 030SJ5i | 0,6 |
| Haemophilus influenzae 351CB12 | 5 |

## Revendications

1. Sous toutes leurs formes stéréoisomères possibles ainsi que leurs mélanges, les composés de formule (I) :

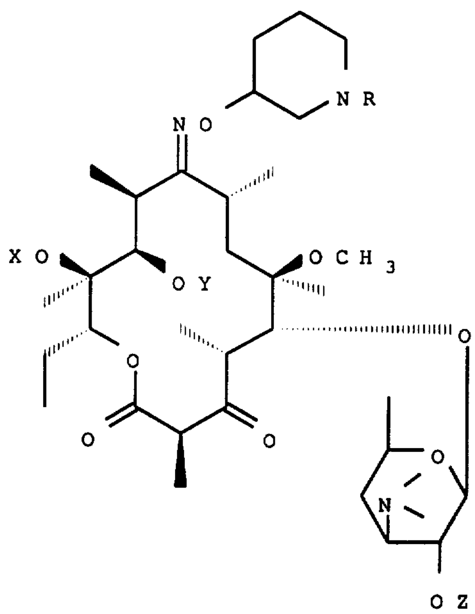

(I)

dans laquelle :

- X et Y représentent un atome d'hydrogène ou forment ensemble un radical :

$$\overset{O}{\overset{\|}{-C-}}$$

caractérisés en ce que

- R représente un radical :

$$-(CH_2)_m-(\underset{A}{\underset{|}{C}}=\underset{B}{\underset{|}{C}})_nX_A$$

dans lequel m représente un nombre entier compris entre 0 et 20, n représente le nombre 0, 1, 2, ou 3, et ou bien A et B identiques ou différents représentent un atome d'hydrogène ou un atome d'halogène ou un radical alkyle ou aryle, renfermant jusqu'à 8 atomes de carbone, la géométrie de la double liaison étant E ou Z ou un mélange E + Z,
ou bien A et B forment une troisième liaison entre les atomes de carbone auxquels ils sont liés,
ou bien $X_A$ représente un radical phényle éventuellement substitué par un ou plusieurs radicaux choisis dans le groupe constitué par les radicaux carboxyle libre, salifié, estérifié ou amidifié, les radicaux hydroxyles, les atomes d'halogène, les radicaux $NO_2$, les radicaux $C{\equiv}N$, les radicaux alkyle, alkényle et alkynyle, O-alkyle, O-alkényle et O-alkynyle, S-alkyle, S-alkényle et S-alkynyle, SO-alkyle, SO-alkényle, SO-alkynyle, $SO_2$-alkyle, $SO_2$-alkényle, $SO_2$-alkynyle, renfermant jusqu'à 12 atomes de carbone éventuellement substitués par un ou plusieurs atomes d'halogène, les radicaux aryle, O-aryle, S-aryle carbocycliques ou hétérocycliques renfermant jusqu'à 14 atomes de carbone, les radicaux :

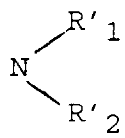

$R'_1$ et $R'_2$ identiques ou différents représentant un atome d'hydrogène ou un radical alkyle, renfermant jusqu'à 12 atomes de carbone, les radicaux aryles eux-mêmes éventuellement substitués par l'un des substituants indiqués ci-dessus comme substituants des radicaux aryles,
ou bien $X_A$ représente un radical :

$$-O-C(C_6H_5)_3$$

dans lequel le ou les radicaux phényle sont éventuellement substitués par un ou plusieurs des substituants indiqués précédemment,
ou bien $X_A$ représente un radical :

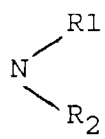

dans lequel $R_1$ et $R_2$ identiques ou différents représentent un atome d'hydrogène ou un radical alkyle renfermant jusqu'à 12 atomes de carbone, ainsi que leurs sels d'addition avec les acides.

**2.** Les composés de formule (I) tels que définis à la revendication 1 dans lesquels n représente le nombre 0 ou 1.

**3.** Les composés de formule (I) tels que définis à la revendication 2 dans lesquels m représente un nombre entier compris entre 2 et 6, ainsi que leurs sels d'addition avec les acides.

**4.** Les composés de formule (I) tels que définis à la revendication 3 dans lesquels $X_A$ représente un radical phényle, éventuellement substitué par un ou plusieurs radicaux choisis dans le groupe constitué par les atomes d'halogène, les radicaux méthyles, trifluorométhyles, hydroxy, méthoxy, phényles et phénoxy éventuellement substitués par un ou plusieurs atomes d'halogène, ainsi que leurs sels d'addition avec les acides.

**5.** Les composés de formule (I) tels que définis à la revendication 4 dans lesquels R représente un radical $-(CH_2)_3Ar$, $-(CH_2)_4-Ar$ ou $-CH_2-CH{=}CH-Ar$, Ar représentant un radical phényle, éventuellement substitué, ainsi que leurs sels d'addition avec les acides.

**6.** Les composés de formule (I) définis à la revendication 1 dans lesquels $X_A$ représente un radical dodécylamino.

**7.** A titre de médicaments les composés de formule I définis à l'une quelconque des revendications 1 à 6, ainsi que leurs sels d'addition avec les acides pharmaceutiquement acceptables.

8. Les compositions pharmaceutiques renfermant comme principe actif au moins un médicament défini à la revendication 7.

9. Procédé de préparation des composés de formule (I) tels que définis à la revendication 1, caractérisé en ce que l'on soumet un composé de formule (II) :

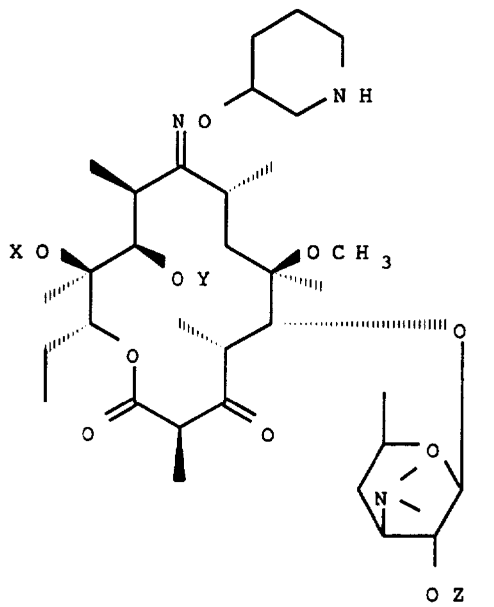

(II)

sous toutes ses formes stéréoisomères possibles ainsi que ses mélanges, dans laquelle X, Y et Z conservent la même signification que dans la revendication 1, à l'action d'un composé de formule (III) :

RCHO (III)

dans laquelle R conserve la même signification que dans la revendication 1, pour obtenir le composé de formule (I) correspondant que l'on soumet, si désiré, à l'action d'un agent d'estérification de la fonction hydroxyle en 2 ou à l'action d'un acide pour en former le sel.

**Patentansprüche**

1. In allen ihren möglichen stereoisomeren Formen sowie deren Gemischen die Verbindungen der Formel (I):

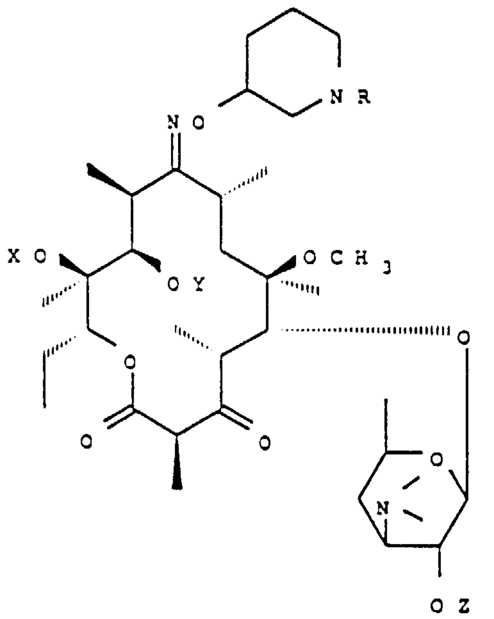

(I)

in der:

- X und Y ein Wasserstoffatom darstellen oder zusammen einen Rest:

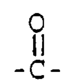

bilden, dadurch gekennzeichnet, daß
- R einen Rest:

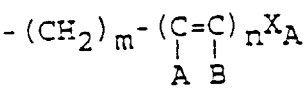

darstellt, in dem m eine ganze Zahl zwischen 0 und 20 darstellt, n die Zahl 0, 1, 2 oder 3 darstellt, und entweder A und B, gleich oder verschieden, ein Wasserstoffatom oder ein Halogenatom oder einen Alkyl- oder Arylrest darstellen, der bis zu 8 Kohlenstoffatome enthält, wobei die Geometrie der Doppelbindung E oder Z oder ein Gemisch E + Z ist,
oder aber A und B eine dritte Bindung zwischen den Kohlenstoffatomen, mit denen sie verbunden sind, bilden, entweder $X_A$ einen Phenylrest darstellt, der gegebenenfalls mit einem oder mehreren Resten substituiert ist, die aus der Gruppe ausgewählt sind, die besteht aus den freien, versalzten, veresterten oder amidierten Carboxylresten, den Hydroxyresten, den Halogenatomen, den Resten $NO_2$, den Resten C≡N, den Alkyl-, Alkenyl- und Alkinyl-, O-Alkyl-, O-Alkenyl- und O-Alkinyl-, S-Alkyl-, S-Alkenyl- und S-Alkinyl-, SO-Alkyl-, SO-Alkenyl-, SO-Alkinyl-, $SO_2$-Alkyl-, $SO_2$-Alkenyl-, $SO_2$-Alkinylresten, die bis zu 12 Kohlenstoffatome enthalten, die gegebenenfalls mit einem oder mehreren Halogenatomen substituiert sind, den carbocyclischen oder heterocyclischen Aryl-, O-Aryl-, S-Arylresten, die bis zu 14 Kohlenstoffatome enthalten, den Resten:

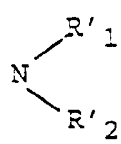

wobei $R'_1$ und $R'_2$, gleich oder verschieden, ein Wasserstoffatom oder einen Alkylrest, der bis zu 12 Kohlenstoffatome umfaßt, die Arylreste, die gegebenenfalls selbst mit einem der oben als Substituenten der Arylreste angegebenen Substituenten substituiert sind, darstellen,
oder aber $X_A$ einen Rest:

$$-O-C(C_6H_5)_3$$

darstellt, in dem der oder die Phenylreste gegebenenfalls mit einem oder mehreren der zuvor angegebenen Substituenten substituiert ist/sind,
oder aber $X_A$ einen Rest:

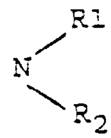

darstellt, in dem $R_1$ und $R_2$, gleich oder verschieden, ein Wasserstoffatom oder einen Alkylrest darstellen, der bis zu 12 Kohlenstoffatome umfaßt, sowie deren Additionssalze mit den Säuren.

**2.** Die Verbindungen der Formel (I), wie in Anspruch 1 definiert, in denen n die Zahl 0 oder 1 darstellt.

**3.** Die Verbindungen der Formel (I), wie in Anspruch 2 definiert, in denen m eine ganze Zahl zwischen 2 und 6 darstellt, sowie deren Additionssalze mit den Säuren.

**4.** Die Verbindungen der Formel (I), wie in Anspruch 3 definiert, in denen $X_A$ einen Phenylrest darstellt, der gegebenenfalls mit einem oder mehreren Resten substituiert ist, die aus der Gruppe ausgewählt sind, die besteht aus den Halogenatomen, den Methyl-, Trifluormethyl-, Hydroxy-, Methoxy-, den Phenyl- und Phenoxyresten, die gegebenenfalls mit einem oder mehreren Halogenatomen substituiert sind, sowie deren Additionssalze mit den Säuren.

**5.** Die Verbindungen der Formel (I), wie in Anspruch 4 definiert, in denen R einen Rest $-(CH_2)_3Ar$, $-(CH_2)_4-Ar$ oder $-CH_2-CH=CH-Ar$ darstellt, wobei Ar einen gegebenenfalls substituierten Phenylrest darstellt, sowie deren Additionssalze mit den Säuren.

**6.** Die in Anspruch 1 definierten Verbindungen der Formel (I), in denen $X_A$ einen Dodecylaminorest darstellt.

**7.** Als Medikamente die Verbindungen der Formel (I), die in einem der Ansprüche 1 bis 6 definiert sind, sowie deren Additionssalze mit den pharmazeutisch annehmbaren Säuren.

**8.** Die pharmazeutischen Zusammensetzungen, die als Wirkstoff wenigstens ein in Anspruch 7 definiertes Medikament enthalten.

**9.** Verfahren zur Herstellung der Verbindungen der Formel (I), wie in Anspruch 1 definiert, dadurch gekennzeichnet, daß man eine Verbindung der Formel (II):

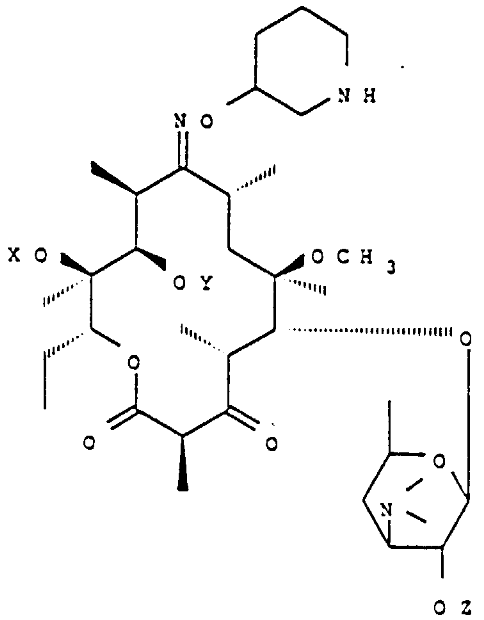

(II)

in allen ihren möglichen stereoisomeren Formen sowie ihren Gemischen, in der X, Y und Z die gleiche Bedeutung wie in Anspruch 1 behalten, der Einwirkung einer Verbindung der Formel (III):

RCHO (III)

in der R die gleiche Bedeutung wie in Anspruch 1 behält, unterzieht, um die entsprechende Verbindung der Formel (I) zu erhalten, die man, wenn gewünscht, der Einwirkung eines Mittels zur Veresterung der Hydroxyfunktion in 2 oder der Einwirkung einer Säure, um daraus das Salz zu bilden, unterzieht.

## Claims

1. In all their possible stereoisomeric forms as well as their mixtures, the compounds of formula (I):

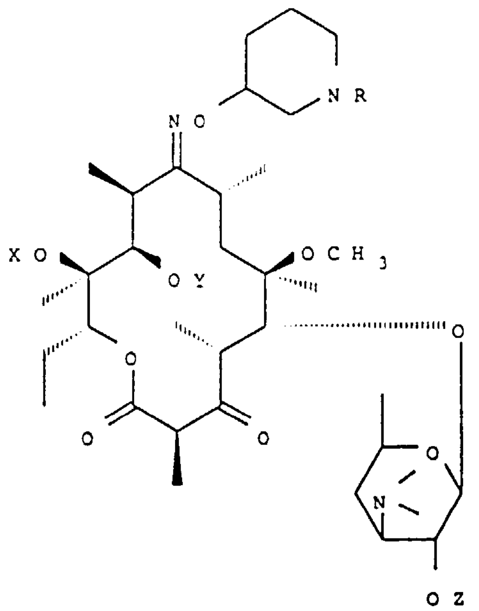

(I)

in which:

- X and Y represent a hydrogen atom or together form a radical:

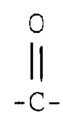

characterized in that

- R represents a radical:

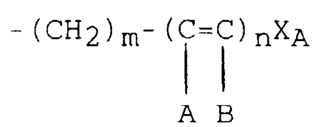

in which m represents an integer comprised between 0 and 20, n represents the number 0, 1, 2, or 3, and either A and B being identical or different represent a hydrogen atom or a halogen atom or an alkyl or aryl radical containing up to 8 carbon atoms, the geometry of the double bond being E or Z or an E + Z mixture, or A and B form a third bond between the carbon atoms to which they are linked, either $X_A$ represents a phenyl radical optionally substituted by one or more radicals chosen from the group constituted by free, salified, esterified or amidified carboxyl radicals, hydroxyl radicals, halogen atoms, the $NO_2$ radicals, the following radicals: C N, alkyl, alkenyl and alkynyl, O-alkyl, O-alkenyl and O-alkynyl, S-alkyl, S-alkenyl and S-alkynyl, SO-alkyl, SO-alkenyl, SO-alkynyl, $SO_2$-alkyl, $SO_2$-alkenyl, $SO_2$-alkynyl, containing up to 12 carbon atoms optionally substituted by one or more halogen atoms, carbocyclic or heterocyclic aryl, O-aryl, S-aryl radicals containing up to 14 carbon atoms, the following radicals:

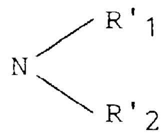

R'$_1$ and R'$_2$ being identical or different and representing a hydrogen atom or an alkyl radical containing up to 12 carbon atoms, the aryl radicals themselves being optionally substituted by one of the substituents indicated above as substituents of the aryl radicals,
or $X_A$ represents a radical:

$$-O-C(C_6H_5)_3$$

in which the phenyl radical(s) are optionally substituted by one or more of the substituents indicated previously,
or $X_A$ represents a radical:

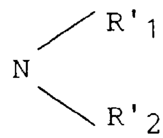

in which $R_1$ and $R_2$ identical or different represent a hydrogen atom or an alkyl radical containing up to 12 carbon atoms, as well as their addition salts with acids.

**2.** The compounds of formula (I) as defined in claim 1 in which n represents the number 0 or 1.

**3.** The compounds of formula (I) as defined in claim 2 in which m represents an integer comprised between 2 and 6, as well as their addition salts with acids.

**4.** The compounds of formula (I) as defined in claim 3 in which $X_A$ represents a phenyl radical, optionally substituted by one or more radicals chosen from the group constituted by halogen atoms, methyl, trifluoromethyl, hydroxy, methoxy, phenyl and phenoxy radicals optionally substituted by one or more halogen atoms, as well as their addition salts with acids.

**5.** The compounds of formula (I) as defined in claim 4 in which R represents a $-(CH_2)_3Ar$, $-(CH_2)_4-Ar$ or $CH_2-CH=CH-Ar$ radical, Ar representing an optionally substituted phenyl radical, as well as their addition salts with acids.

**6.** The compounds of formula (I) defined in claim 1 in which $X_A$ represents a dodecylamino radical.

**7.** As medicaments, the compounds of formula (I) defined in any one of claims 1 to 6, as well as their addition salts with pharmaceutically acceptable acids.

**8.** The pharmaceutical compositions containing at least one medicament defied in claim 7 as active ingredient.

**9.** Preparation process for the compounds of formula (I) as defined in claim 1, characterized in that a compound of formula (II):

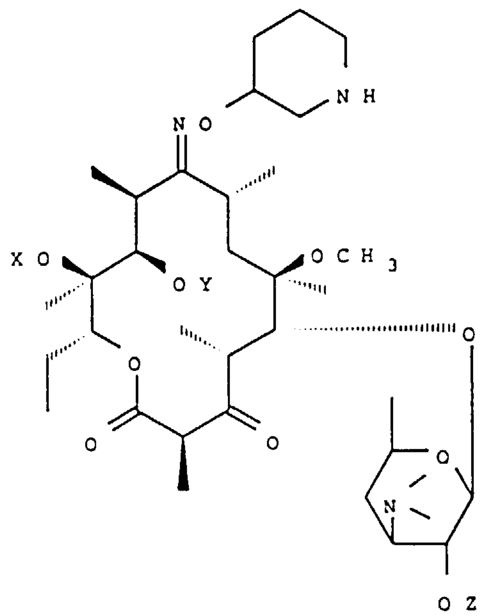

(II)

in all its possible stereoisomeric forms as well as its mixtures, in which X, Y and Z keep their previous meaning, is subjected to the action of a compound of formula (III):

RCHO (III)

in which R keeps the same meaning as in claim 1, in order to obtain the corresponding compound of formula (I) which is subjected, if desired, to the action of an esterification aqent of the hydroxyl function in position 2 or to the action of an acid in order to form the salt.